(19) Europäisches Patentamt
European Patent Office
Office européen des brevets

(11) EP 2 854 858 B1

(12) EUROPEAN PATENT SPECIFICATION

(45) Date of publication and mention
of the grant of the patent:
**10.12.2025 Bulletin 2025/50**

(21) Application number: **13726003.0**

(22) Date of filing: **31.05.2013**

(51) International Patent Classification (IPC):
***A61K 47/34*** (2017.01)

(52) Cooperative Patent Classification (CPC):
**A61K 9/0024; A61K 31/519; A61K 47/34;
A61P 25/18**

(86) International application number:
**PCT/EP2013/061320**

(87) International publication number:
**WO 2013/178812 (05.12.2013 Gazette 2013/49)**

(54) **RISPERIDONE IMPLANT FORMULATION**

RISPERIDON- IMPLANTATFORMULIERUNG

FORMULATION POUR IMPLANT CONTENANT DE LA RISPÉRIDONE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **31.05.2012 EP 12170362**

(43) Date of publication of application:
**08.04.2015 Bulletin 2015/15**

(73) Proprietor: **Laboratorios Farmacéuticos Rovi,
S.A.
28037 Madrid (ES)**

(72) Inventors:
• **GUTIERRO ADURIZ, Ibon
E-28037 Madrid (ES)**
• **FRANCO RODRÍGUEZ, Guillermo
E-28037 Madrid (ES)**

(74) Representative: **Elzaburu S.L.P.
Edificio Torre de Cristal
Paseo de la Castellana 259 C, planta 28
28046 Madrid (ES)**

(56) References cited:
EP-A1- 2 529 757     EP-A2- 2 529 756
WO-A1-2011/151355     WO-A2-2011/151356

Remarks:
The file contains technical information submitted
after the application was filed and not included in this
specification

## Description

### TECHNICAL FIELD

The present invention relates to pharmaceutical compositions for intramuscular injection comprising the drug risperidone and/or ist pharmaceutically acceptable salts, wherein the composition releases the drug with an immediate onset of action and continuously for at least 4 weeks, and wherein the composition has a pharmacokinetic profile *in vivo* that makes it suitable to be administered each 4 weeks or even longer periods. Specifically, the present invention is related to compositions for injectable *in-situ* forming biodegradable implants comprising risperidone.

### BACKGROUND ART

Risperidone and paliperidone are atypical antipsychotic drugs with benzisoxazole and piperidine functional groups, which act as strong dopaminergic antagonist and selective serotonin receptor antagonist. Risperidone is FDA approved for the treatment of schizophrenia since 1993. It is the only drug presently approved for the treatment of schizophrenia in young people under 18 years, and together with lithium, for the treatment of bipolar disorders in children/youth ages between 10-18 years old. Conventional risperidone therapy of schizophrenia involves daily oral tablets, although it is also available as a solution and orally disintegrating tablets.

In fact, one of the intrinsic problems that risperidone or paliperidone-targeted patients usually face is the dissociation of some schizophrenic patients from the treatment, moreover when it consists of a daily medication, leading to irregular or inconstant treatments and favouring the appearance of psychotic crisis. Moreover, this kind of therapy gives rise to high differences in the plasma levels (measured as the difference between Cmax and Cmin) in patients, therefore usually affecting the patient's mood.

Risperidone and paliperidone are therefore good drug candidates for incorporation into sustained delivery devices, where the patients would be covered or treated for long time periods with just one dose and without the need of caregivers to pay attention to a daily medication, and where more homogeneous plasma levels in the patient are desirable. Other indications may involve bipolar mania and schizoaffective disorder, and its possible use in autism and Asperger's syndrome and Tourette's disorder may be of benefit to the patients.

Risperidone was initially marketed as Risperdal® and recently became generic. Currently, the long-acting injectable risperidone formulation, Risperdal Consta®, is the first depot atypical antipsychotic drug in the market. It is an intramuscular risperidone-containing PLGA microparticles formulation and is intended to deliver therapeutic levels of risperidone by bi-weekly administrations. However, due to the inherent lag phase of most microparticle based products, the patient is required to supplement the first weeks with daily doses of oral risperidone after first administration. Approximately three weeks after a single intramuscular injection of Risperdal Consta® and concurrent daily doses of oral risperidone, the microspheres release sufficient risperidone in the systemic circulation that the patient can discontinue supplementation with daily doses of the oral therapy. However, this period of oral supplementation could be a risk factor of non-compliance. Also, the presence on the body of two doses at the same time could be a potential risk of adverse events, such as irregular formulation behaviour and toxicity.

Paliperidone recently received marketing approval as the first oral atypical antipsychotic with an extended release, which is achieved by an osmotic-controlled release oral delivery system. Paliperidone ER (WO2006/17537) is marketed as Invega Sustenna® and unsaturated derivatives thereof are described in WO2008/128436. Other extended release oral dosage forms for paliperidone are under development. Due the presence of a secondary hydroxyl group, paliperidone may be provided as a prodrug. WO2009/15828 details acid-labile low molecular weight prodrugs of paliperidone intended to undergo hydrolysis in the stomach.

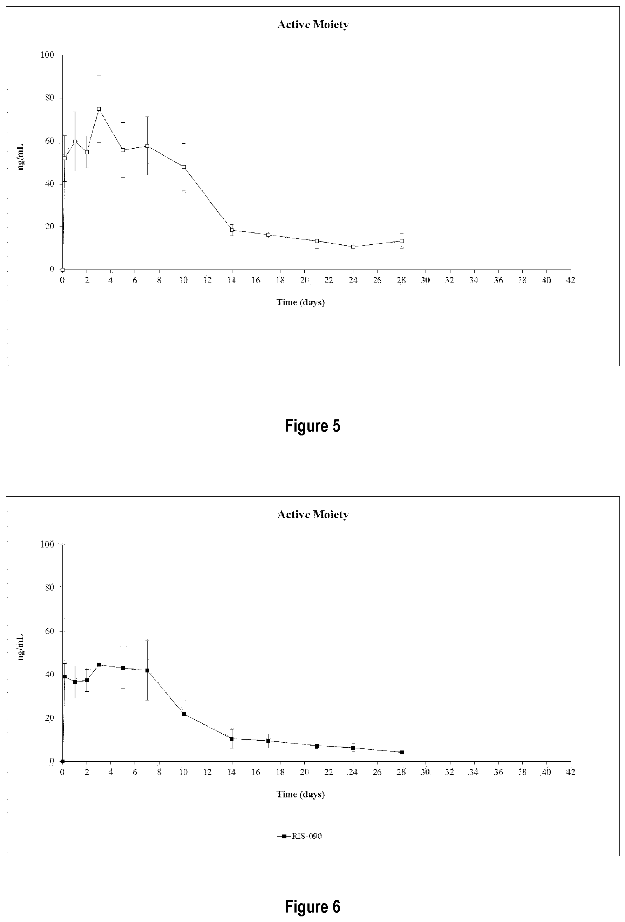

Therefore, in view of the state of the art, it is of interest to develop very long-acting, injectable depots of risperidone. There is great need to improve the compliance factor particularly in the treatment of schizophrenia. The development of once-weekly or even longer acting injectable depot formulations of those drugs will mark a significant step forward to ensure continuous and steady supply of the effective medication. In US5965168 application is described compounds of formula I which are formulated in sustained release microparticles. Risperidone is mentioned as the preferred compound and risperidone is used as basis for all experiments therein. Fig 5 therein shows the plasma concentration time curves for the active moiety (sum of risperidone and paliperidone) after intramuscular injection of risperidone depot.

WO02008/153611 describes sustained release formulations of risperidone and metabolites. Here, risperidone is mixed with a soluble thermoplastic polymer, forming an encapsulating residue upon injection from which risperidone is slowly released. EP2234617 reveals ester-linked prodrugs of paliperidone. The substance paliperidone palmitate is approved as a once-monthly atypical antipsychotic intramuscular injection for treating schizophrenia and preventing recurrence of its symptoms. Paliperidone palmitate is formulated in a submicrocrystalline form. Paliperidone palmitate due to its dissolution rate-limited absorption exhibits flip-flop kinetics, where the apparent half-life is controlled by the absorption rate constant. Additionally the volume of injected drug product also impacts the apparent rate constant. It was also discovered that deltoid injections result in a faster rise in initial plasma concentration, facilitating a rapid attainment of potential therapeutic concentrations. Consequently, to facilitate patients' attaining a rapid therapeutic concentration of paliperidone it is preferred to provide the initial loading dose of paliperidone palmitate in the deltoids. The loading dose should be from about 100 mg-eq. to about 150 mg-eq. of paliperidone provided in the form of paliperidone palmitate. After the first or more preferably after the second loading dose injection patients will be approaching a steady state concentration of paliperidone in their plasma and may be injected in either the deltoid or the gluteal muscle thereafter. However, it is preferred that the patients receive further injections in the gluteal muscle. US2009/163519 outlines corresponding dosing regimen for long-acting injectable paliperidone esters of the palmitate type.

Other antipsychotic depot medications are also characterized by the need for concomitant oral medication or booster injections in order to obtain desired plasma levels of the active drug. For example, Risperdal Consta® requires oral antipsychotic treatment during the initiation phase.

Other depot formulation is described by the international application WO2011/42453. This specification describes a pharmaceutical composition for subcutaneous injection comprising a paliperidone compound. In particular the composition refers to a composition in which paliperidone is linked by an ester bondage to a hydrogel. The formulation releases paliperidone by ester bondage cleavage and claims to release the paliperidone with an immediate onset of action and for an extended release time. Moreover, this specification relates to a pharmaceutical composition for subcutaneous injection comprising a paliperidone compound in a certain concentration.

Finally, another antipsychotic injectable depot composition is described in the international application number WO2011/151355. This application is directed to a composition that can be used to deliver an antipsychotic drug such as risperidone as an injectable in-situ forming biodegradable implant for extended release providing therapeutic plasma levels from the first day. The composition is in the form of drug suspension on a biodegradable and biocompatible copolymer or copolymers solution using water miscible solvents that is administered in liquid form. Once the composition contacts the body fluids, the polymer matrix hardens retaining the drug, forming a solid or semisolid implant that releases the drug in a continuous manner.

## SUMMARY OF THE INVENTION

Therefore, the compositions already described in the state of the art do not cover the existing needs in risperidone compositions, kits and treatments for psychiatric disorders, and there still exists a need of compositions and devices to allow a controlled, constant release of the drug during prolonged periods of time during at least 4 weeks without a concomitant treatment or initial doses of risperidone.

The solution to this necessity is provided by an injectable intramuscular depot composition suitable for forming an in situ solid implant in a body, comprising a drug which is risperidone and/or its salts in any combination thereof, a biocompatible copolymer based on lactic and glycolic acid having a monomer ratio of lactic to glycolic acid in the range of 45:55 to 55:45 and preferably about 50:50 and a DMSO solvent, wherein the composition releases the drug with an immediate onset of action and continuously for at least 4 weeks and wherein the composition has a pharmacokinetic profile in vivo with substantially no burst release of the drug characterised in that the biocompatible copolymer has a molecular weight between 30 and 46 and preferably between 30 and 36 kDa and has an inherent viscosity in the range of 0.26-0.31 and preferably between 0.26-0.29 dl/g $\pm$ 10%.

The scope of the invention is defined by the claims.

## DETAILED DESCRIPTION OF THE INVENTION

The compositions of the invention comprise at least a polymer or polymer matrix, a solvent and a drug.

The **polymer** or polymer matrix is preferably a biocompatible and biodegradable polymer matrix. In order not to cause any severe damage to the body following administration, the preferred polymers are biocompatible, non-toxic for the human body, not carcinogenic, and do not induce significant tissue inflammation. The polymers are preferably biodegradable in order to allow natural degradation by body processes, so that they are readily disposable and do not accumulate in the body. The preferred polymeric matrices in the practice in this invention are selected from end-capped terminal carboxylic poly-lactide and poly-glycolic acid copolymers mixed in a ratio ranging from 45:55 to 55:45, and preferably of about 50:50, with an average molecular weight in the range of 30-45 and preferably in the range of 30-36 KDa, and an inherent viscosity preferably in the range of 0.25-0.31 and more preferably in the range of 0.26-0.29 dl/g $\pm$10%.

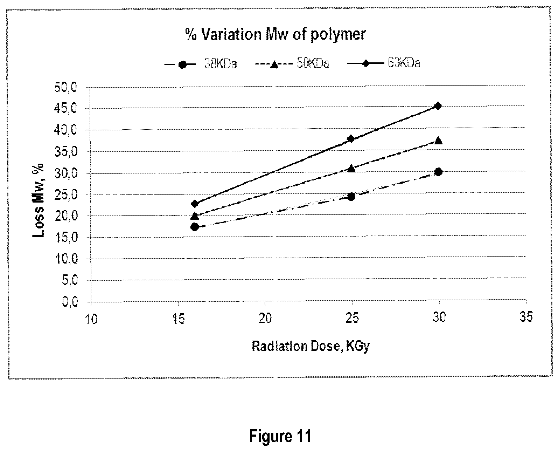

A commercial polymer with the required molecular weight can certainly be used. However, we have determined that the essential range of its molecular weight is between 30-46 and preferably between 30-45 kDa. Additionally we have determined in an in-house custom design that the molecular weight of the polymer can be varied by irradiating it with a radiation dose of between 15 and 30 kGy $\pm$10% or even more at a temperature lower than 8°C and this was not obvious in view of the state of the art to the skilled person (see Fig. 11). For example, the molecular weight of a commercially available polymer in a certain moment can be 50 KDa as an average value. We have determined a method for varying this molecular weight by irradiating the polymer with a certain dose of radiation that can be previously calculated. If done under controlled conditions, it is possible to obtain a mathematical model showing that the molecular weight of the polymer can be decreased with increasing irradiation doses. Since, if the molecular weight of the polymer is adjusted, its inherent viscosity is correspondingly varied, it follows that by irradiating the polymer with certain defined radiation doses we achieve both the adjustment of its molecular weight and its inherent viscosity.

Therefore, for example:

- When we need to use a PLGA polymer having a molecular weight between 30 and 46 kDa and an inherent viscosity value in the range of 0.25-0.31 dl/g, and we have as the starting polymer a polymer with 56 KDa of average molecular weight, we have determined that a radiation dose of 25 KGy is required to reduce its molecular weight to the cited range of 30-46 kDa.

- When we need to use a PLGA polymer having a molecular weight between 30 and 40 and preferably between 30 and 36kDa, and an inherent viscosity value in the range of 0.25-0.31 dl/g and preferably 0.26-0.29 dl/g, and we have as the starting polymer a polymer with 50 KDa of average molecular weight, we have determined that a radiation dose of 25 KGy is required to reduce its molecular weight to the cited range of 30-40 kDa and preferably 36-40 kDa.

- When we need to use a PLGA polymer having a molecular weight between 30 and 40 kDa and an inherent viscosity value in the range of 0.25-0.31 dl/g, and we have as the starting polymer a polymer with 38 KDa of average molecular weight, we have determined that there is no need to use any radiation dose.

- When we need to use a PLGA polymer having a molecular weight between 30 and 36 kDa and an inherent viscosity value in the range of 0.25-0.31 and preferably of 0.26-0.29 dl/g, and we have as the starting polymer a polymer with 38 KDa of average molecular weight, we have determined that a radiation dose of 16 KGy is required to reduce its molecular weight to the cited range of 30-36 kDa.

- When we need to use a PLGA polymer having a molecular weight between 30 and 36 kDa and an inherent viscosity value in the range of 0.25-0.31 and preferably of 0.26-0.29 dl/g and we have as the starting polymer a polymer with 31 KDa of average molecular weight, we have determined that there is no need to use any radiation dose.

- When we need to use a PLGA polymer having a molecular weight between 30 and 46 kDa and an inherent viscosity value in the range of 0.25-0.31 and preferably of 0.26-0.29 dl/g, and we have as the starting polymer a polymer with 63 KDa of average molecular weight, we have determined that a radiation dose of 30 KGy is required to reduce its molecular weight to the cited range of 30-46 kDa and preferably of 30-36 kDa.

In these experimental tests, the temperature conditions for the polymer during the irradiation were about 8°C. However, other temperatures can be used, such as e.g. lower than 35°C, or lower than 25°C, although in these cases the relationships between the radiation dose and the resulting molecular weight may vary.

The procedure is especially suitable for the manufacturing of the compositions described in the present invention. Furthermore, the filling of the solid polymer into syringes represents a real challenge in the manufacturing of injectable formulations. The polymer, manufactured as a non-sterile product, requires undergoing sterilization in order to achieve a formulation that can be injected into human beings. Probably the best way to solve this technical issue is to subject the polymer to sterilization by gamma or beta irradiation. Irradiation represents a challenging issue when using biodegradable polymers, as irradiation can disrupt the chains into fractions of smaller size. Control of the polymer molecular weight appears as again as the critical parameter to control the final characteristics of a product after a sterilization process.

As previously explained, chain size reduction by irradiation can be mathematically modelled or controlled in order to predict the final molecular weight of a polymer to be used as raw material having a molecular weight higher than desired. Therefore, once determined the fill weight of the polymer to be filled in a container (for example, the fill weight of the polymer in a syringe) and the bio-burden present in the polymer as raw material, the irradiation dose required to get the polymer sterile (as specified by ISO 11137) is selected for the required fill weight. Then the mathematical model describing the loss of molecular weight for a certain polymer versus the irradiated dose can identify the initial molecular weight of the polymer to be used as raw material required obtaining, after the irradiation process, a polymer with the desired final molecular weight for the formulation. As the availability of a polymer with a specific molecular weight can be somewhat limited, then we can alternatively select an available polymer with a molecular weight that is higher to what is required according to the irradiation dose identified, and then adjust the irradiation dose to a higher value in order to obtain a sterile polymer with the required molecular weight.

The concentration of the polymeric component in the compositions of the invention is preferably comprised in the range of 24-50%, (expressed as the percentage of polymer weight based on total composition weight) and more preferably 25-27%.

For the purpose of the present invention, throughout the present specification the term intrinsic or inherent viscosity ($\eta_{inh}$) of the polymer is defined as the ratio of the natural logarithm of the relative viscosity, $\eta_r$, to the mass concentration of the polymer, c, i.e.:

$$\eta_{inh} = (\ln \eta_r)/c$$

and the relative viscosity ($\eta_r$) is the ratio of the viscosity of the solution $\eta$ to the viscosity of the solvent $\eta_s$, i.e.:

$$\eta_r = \eta/\eta_s$$

If not otherwise specified, the intrinsic viscosity and molecular weight values throughout the present specification are to be understood as measured with the method explained in example 1. The value of intrinsic viscosity is considered in the present specification, as commonly accepted in the art, as an indirect indicator of the polymer molecular weight. In this way, a reduction in the intrinsic viscosity of a polymer, measured at a given concentration in a certain solvent, with same monomer composition and terminal end groups, is an indication of a reduction in the polymer molecular weight (IUPAC. Basic definitions of terms relating to polymers 1974. Pure Appl. Chem. 40, 477-491 (1974).

The preferred **solvents** are non-toxic, biocompatible and appropriate for parenteral injection. Solvents susceptible of causing toxicity should not be used for the injection of any material into any living body. More preferably, selected solvents are biocompatible in order not to cause any severe tissue irritation or necrosis at the injection site. Therefore, the solvent is preferably classified as class II or III, and more preferably class III, according to ICH Guidelines. For the formation of the *in-situ* implant, the solvent should preferably diffuse quickly from the polymeric solution towards surrounding tissues when is exposed to physiological fluids. Consequently, the solvent is preferably DMSO.

The **drug** is preferably risperidone, and/or paliperidone and all pharmaceutically acceptable salts or combinations thereof. This drug is preferably at least partly suspended in the solvent. The solubility of the drug in the solvent is preferably lower than 90 mg/ml, more preferably lower than 65 mg/ml, and most preferably below 10 mg/ml. The advantage of this low solubility is that the initial burst of the drug when the solvent diffuses to the external aqueous medium is greatly reduced. In addition, in the final compositions of the invention the drug is provided in a preferred concentration between 4 and 16 wt%, expressed as the percentage of the drug in respect of the total composition weight. More preferably, the drug content is between 7 and 15%, and most preferably about 13% in respect of the total composition weight.

The expression "about 50:50" as used in this description, refers to a monomer ratio of lactic to glycolic acid of biocompatible copolymer based on lactic and glycolic acid it is applied in the context of the invention for a monomer ratio measure with an standard technical error of ± 10% .

One of the factors contributing to control the initial release of the composition of the invention is the viscosity of the polymeric solution. The "polymeric solution", which is defined as the combination of the polymer and the solvent where it is dissolved, has a preferred viscosity in the range of 1.5-2.1 Pa.s, more preferably between 1.6-1.9 Pa.s and even more preferably between 1.7-1.8 Pa.s ±10%.

A second factor contributing to control the initial release of the compositions of the invention is the biocompatible copolymer molecular weight that must be between 30 and 46 and preferably between 30 and 45 kDa. The adequate balance in this composition between drug solubility in the solvent and the molecular weight of the polymer in the implant (that controls the polymer precipitation process and the final structural characteristics of the implant) allows the formulation to limit the amount of risperidone that can be released in the solvent diffusion phase after the intramuscular injection. Once the formulation is injected in the intramuscular tissue, the DMSO is rapidly dissolved in the surrounding aqueous environment. The relative increase of the polymer concentration in DMSO over the polymer solubility in the solvent leads to the formation of a polymer precipitate that entraps the risperidone that was not solubilized in the solvent. Molecular weight of the polymer has a great impact in this critical step, as too low weighed chains have delayed precipitation time compared to the chains having the weight in the adequate range. This delayed precipitation allows the drug to increase contact with the surrounding fluids towards the drug is being released.

Therefore, low molecular weighed chains lead to an excessive release of risperidone after the injection and potentially to obtain toxic plasma levels on the first days after the injection. Molecular weight of the polymer also can affect the release of the drug from the intramuscularly injected implant after solvent diffusion and polymer precipitation. Molecular weights over the specified range are not capable to maintain adequate release rates of risperidone by diffusion.

Additionally, higher molecular weight chains in the intramuscular tissue require longer hydrolysis times in order to provide soluble fractions that could release the drug entrapped in the polymer matrix. A higher remaining drug content to be released could lead to the obtention of undesirably high active moiety plasma values, or plasma values after 30 days post injection that could somehow interfere with the following dose as the formulation is intended to be injected several times into the human, each 4 weeks or 30 days.

One important aspect of this invention is an injectable intramuscular depot composition suitable for forming an *in situ* solid implant in a body, comprising a drug which is risperidone or any pharmaceutically acceptable salt thereof in any combination, a biocompatible copolymer based on lactic and glycolic acid having a monomer ratio of lactic to glycolic acid of ranging from 45:55 to 55:45 and preferably about 50:50, and a DMSO solvent, wherein the composition releases the drug with an immediate onset of action and continuously for at least 4 weeks and wherein the composition has a pharmacokinetic profile *in vivo* that makes it suitable to be administered each 4 weeks or even longer periods characterised in that the biocompatible copolymer has a molecular weight between 30 and 46 kDa, preferably between 30 and 36 kDa, has an inherent viscosity in the range of 0.25-0.31 and preferably of 0.26-0.29 dl/g $\pm$10%.

In a preferred embodiment of the invention, the biocompatible copolymer is gamma or beta irradiated in the dose range of 15-30 KGy and at a temperature higher than -40°C but lower than 35°C, more preferably lower than 25°C, even more preferably lower than 15°C and most preferably about 8°C, for adjusting its molecular weight and viscosity ranges.

In a preferred embodiment of the invention, this composition has the particle size distribution of the drug as follows:

- less than 10% particles smaller than 10 microns;
- less than 10% particles larger than 225 microns, and
- a d0.5 value in the range of 40-130 microns.

If not otherwise specified, the particle size distribution was determined by light scattering technique using laser diffractor in wet mode. It is known that particle size distribution results can be altered as a function of the material treatment such the use of high concentrate surfactant agents and/or strong force energies (vortex, sonication, etc). If nothing else is mentioned, drug is not treated and samples are prepared by direct addition to the tank under moderate stirring (2000-3500 rpm). The methodology applied on present invention to determine the drug particle size distribution mimics in a more faithfully way the behaviour of the drug powder on the injectable formulation herein described than other methods which apply force energies to the sample and/or use high concentrate surfactant agents for preparing the samples in order to achieve high degrees of powder disaggregation that cannot be simulated during the manual reconstitution process of the formulation.

And the drug / polymer+drug mass ratio is about 33%, and the content of drug is about 13% w/w of total formulation and viscosity of solution formed between polymer and DMSO is in the range of 1.5-2.1 and preferably of 1.7 - 1.8 P.a.s $\pm$10%.

According another embodiment, the biocompatible copolymer of this invention is gamma or beta radiated, preferably in the range of 10-30 KGy, more preferably in the range of 15-30 KGy, and most preferably between 16 -25 KGy $\pm$10%.

According to another embodiment, the composition is a sterile composition and is suitable for the treatment of schizophrenia or bipolar disorders in the human body.

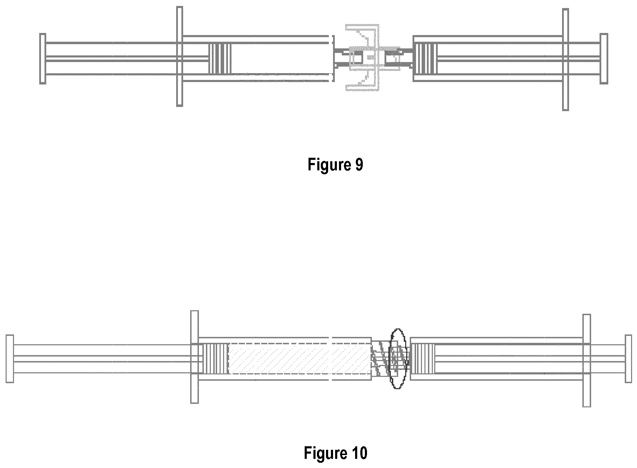

In yet another embodiment, the invention provides a pharmaceutical kit suitable for the in situ formation of a biodegradable implant in a body comprising the composition claimed, wherein the drug and the biocompatible polymer are contained in a first container, and the solvent is contained in a second, separate container. Preferably, at least one of the first and second containers is a syringe, a vial, a device or a cartridge, either disposable or not and more preferably both the first and the second containers are disposable syringes. This aspect of the invention is directed to a kit comprising a first container, preferably syringes, vials, devices or cartridges, all of them either being disposable or not, containing a polymer in solid form, such as PLGA and a drug in the appropriate amounts and a second container, likewise preferably syringes, vials, devices or cartridges, all of them being either disposable or not, containing the water-miscible solvent. When required, the contents of both containers are combined, for example through a connector or by using male-female syringes, and mixed each other so that the compositions according to the invention are reconstituted, for example by moving forwards and backwards the plungers of the syringes. Illustrative preferred embodiments are shown in Figure 9 (syringes connected through a connector device) and in Figure 10 (syringes connected through a direct thread).

According another aspect the invention provides a method for the manufacturing of a composition according to claims, comprising the step of providing a biocompatible copolymer having a polymer weight higher than required for the intramuscular depot composition and then reducing its molecular weight to between 30 and 46 kDa by irradiating it with gamma or beta radiation in the dose range of 15-30KGy.

In a preferred embodiment of this method, when the biocompatible polymer has an initial molecular weight of about 56 kDa, it is irradiated with a radiation dose of about 25 KGy to reduce its molecular weight to between 30 and 46 kDa $\pm$10%.

In a preferred embodiment of this method, when the biocompatible polymer has an initial molecular weight of about 50 kDa, it is irradiated with a radiation dose of about 25 KGy to reduce its molecular weight to between 30 and 40 and preferably between 30 and 36 kDa $\pm 10\%$.

In another embodiment, when the biocompatible polymer has an initial molecular weight of about 38 kDa, it is irradiated with a radiation dose of about 16 KGy to reduce its molecular weight to between 30 and 36 kDa.

In yet another embodiment, when the biocompatible polymer has an initial molecular weight of about 63 kDa, it is irradiated with a radiation dose of about 30 KGy to reduce its molecular weight to between 30 and 46 and preferably between 30 and 36 kDa $\pm 10\%$.

In a preferred embodiment, the injectable depot composition is sterile as a finished product. In other preferred embodiment, the biocompatible polymer is sterilized previously to its aseptic filling process, preferably by irradiation in the range 15-30KGy or by other process like filtration.

Within the context of the present invention, without limitation and in connection with the examples, all technical parameters are considered to have a standard technical measure error of $\pm 10\%$.

The composition according to the invention is for use in the treatment of schizophrenia or bipolar disorder in the human body and is characterized by the administration of:

- A first intramuscular dose of the formulation at day 0, in a dose between 75 to 200 mg, followed by a second dose between days 28-35 with a dose in the range of 25 to 200 mg, and then subsequent 4 weekly doses of the formulation.

## BRIEF DESCRIPTION OF THE FIGURES

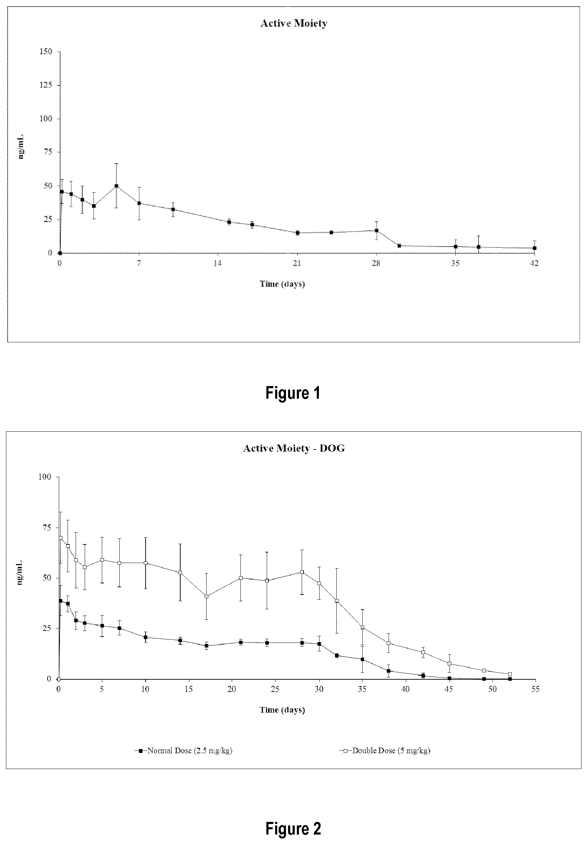

Fig 1.- Active moiety levels profile in dog after the administration of the risperidone formulation described in example 1 to Beagle dogs (n=3). Dose is 2.5 mg/kg. Results are expressed as ng/ml plasma values of active moiety versus time. The table describes Area Under the Curve (AUC) of active moiety plasma levels versus time. AUC all as well as AUC vs three different time frames are included. Units are expressed in h*ng/ml.

Fig 2.- Active moiety levels profile in dog after the administration of the risperidone formulation described in example 2 to two cohorts of Beagle dogs (each cohort n=6). Doses were 2.5 and 5 mg/kg. Results are expressed as ng/ml plasma values of active moiety versus time. The table describes Area Under the Curve (AUC) of active moiety plasma levels versus time. AUC all as well as AUC vs three different time frames are included. Units are expressed in h*ng/ml.

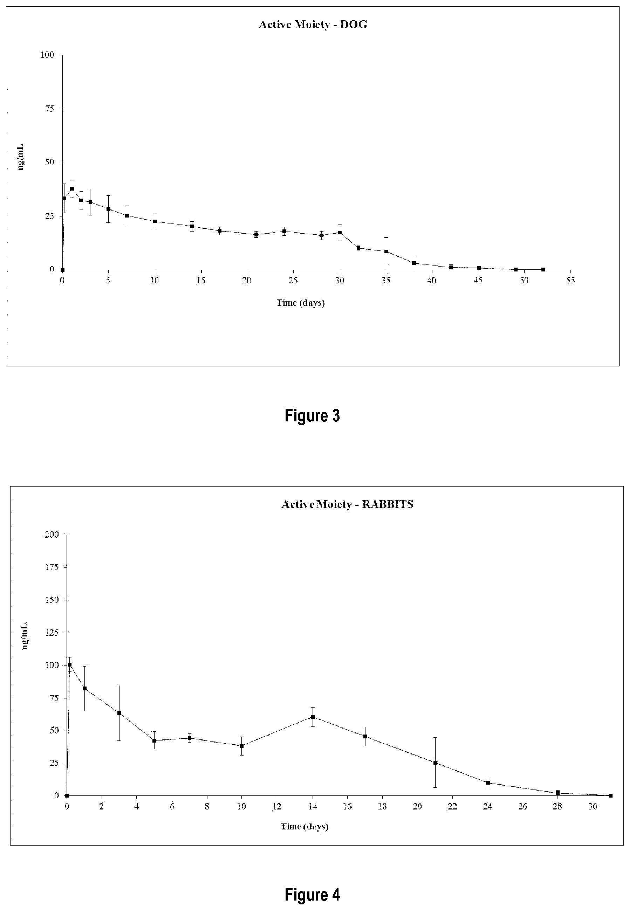

Fig 3.- Active moiety levels profile in dog after the administration of the risperidone formulation described in example 3 to Beagle dogs (n=3). Dose is 2.5 mg/kg. Results are expressed as ng/ml plasma values of active moiety versus time. The table describes Area Under the Curve (AUC) of active moiety plasma levels versus time. AUC all as well as AUC vs three different time frames are included. Units are expressed in h*ng/ml.

Fig 4.- Active moiety levels profile in rabbit after the administration of the risperidone formulation described in example 4 to White New Zealand rabbits (n=3). Dose is 5 mg/kg. Results are expressed as ng/ml plasma values of active moiety versus time. The table describes Area Under the Curve (AUC) of active moiety plasma levels versus time. AUC all as well as AUC vs three different time frames are included. Units are expressed in h*ng/ml.

Fig 5.- Active moiety levels profile in dog after the administration of the risperidone formulation described in example 5 to Beagle dogs (n=3). Dose is 2.5 mg/kg. Results are expressed as ng/ml plasma values of active moiety versus time. The table describes Area Under the Curve (AUC) of active moiety plasma levels versus time. AUC all as well as AUC vs three different time frames are included. Units are expressed in h*ng/ml.

Fig 6.- Active moiety levels profile in dog after the administration of the risperidone formulation described in example 6 to Beagle dogs (n=3). Dose is 2.0 mg/kg. Results are expressed as ng/ml plasma values of active moiety versus time. The table describes Area Under the Curve (AUC) of active moiety plasma levels versus time. AUC all as well as AUC vs three different time frames are included. Units are expressed in h*ng/ml.

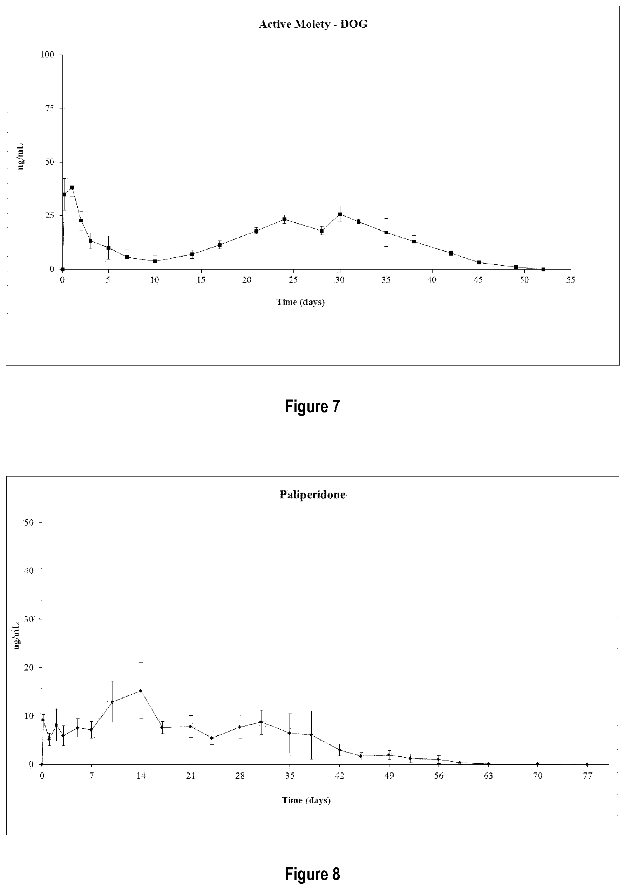

Fig 7.- Active moiety levels profile in dog after the administration of the risperidone formulation described in example 7 to Beagle dogs (n=3). Dose is 2.0 mg/kg. Results are expressed as ng/ml plasma values of active moiety versus time. The table describes Area Under the Curve (AUC) of active moiety plasma levels versus time. AUC all as well as AUC vs three different time frames are included. Units are expressed in h*ng/ml.

Fig 8.- Paliperidone levels profile in dog after the administration of the paliperidone formulation described in example 8 to Beagle dogs (n=3). Dose is 1.5 mg/kg. Results are expressed as ng/ml plasma values of paliperidone versus time.

The table describes Area Under the Curve (AUC) of paliperidone plasma levels versus time. AUC all as well as AUC vs three different time frames are included. Units are expressed in h*ng/ml.

Fig 9.- Drawing of a kit suitable for the preparation of risperidone and paliperidone compositions comprising two male syringes linked by a connector. Polymer+risperidone are contained in one syringe and DMSO filled in the second syringe

Fig 10.- Drawing of a kit suitable for the preparation of risperidone and paliperidone compositions comprising a female syringe linked to a male syringe. Polymer+risperidone can be contained in one syringe and DMSO filled in the second syringe. Preferably female syringe contains polymer+risperidone as a solids and male syringe is filled with DMSO.

Fig 11.- Loss of molecular weight percentage in the custom design. The molecular weight of the polymer can be varied by irradiating it with a certain radiation dose. The table describes the percentage of loss of polymer weight versus radiation dose.

## EXAMPLES

The following examples illustrate the invention and should not be considered in a limitative sense thereof.

In the sense of the present invention, without limitation and in connection with the *in vivo* examples, for "Initial Burst" or initial release it is meant the addition of the plasma levels of risperidone plus those of 9-OH-risperidone, which addition is also called "the active moiety" throughout the present specification, from the moment of the injection until the third day after the administration. In a similar way, an "adequate plasma level profile" in Beagle dog is considered as not more than the 35% of the AUC of the active moiety (risperidone + 9-OH risperidone plasma concentrations) occurring between the moment of the injection until day 7 (included), between 35% and 45% of the AUC of the active moiety occurring from day 7 and until 21 (included), and not more than 45% of the AUC of the active moiety occurring after day 21.

These percentages represent an adequate balance between the different periods in which risperidone is being released from the implant in order to have a formulation to be injected each 4 weeks or each 30 days capable to obtain therapeutic plasma levels of active moiety in a human since the first day of the injection, capable to obtain the desired average active moiety plasma concentrations during the period between injections and with reduced peak-valley plasma values of active moiety that could lead to toxicity or lack of efficacy. Also in the sense of this invention, without limitation and in connection with the examples, acceptable plasma levels of active moiety during the initial burst phase are below 75 ng/ml in Beagle dogs when doses administered are 2.5 mg/kg risperidone.

### Example 1: Depot formulation with Resomer® 503 without radiation

In the present example, the following formulation was prepared:

| | Ingredient | Amount (mg) |
|---|---|---|
| Female 2,25 ml syringe | Lactic-co-glycolic acid copolymer (N-capped) with 50% content of each of the two organic acid monomers and a molecular weight of 32 KDa. | 50 |
| | Risperidone | 25 |
| Male 2,25 ml syringe | Ingredient | Amount (mg) |
| | Dimethyl sulfoxide | 117 |

Risperidone particle size was characterized by light scattering and provided the following distribution of particle size: $d(0,1) = 27.49 \, \mu m$, $d(0,5) = 79.90 \, \mu m$ and $d(0,9) = 176.66 \, \mu m$.

Polymer has been characterized for its molecular weight according to the following technique:

Equipment

GPC chromatograph with triple detector (laser diffraction, viscosimetry, refraction index)

- Viscotek® GPCmax VE 2001 GPC SOLVENT/SAMPLE MODULE
- Viscotek® TDA 305 TRIPLE DETECTOR ARRAY

Reagents:

- Tetrahydrofurane (THF) grade GPC stabilized with butyl hydroxyl toluene (BHT) 250 ppm
- Polystyrene narrow standard (preferably about a molecular weight of 90 or 99 KDa)

Sample preparation:

- 1-2 mg/ml Standard Sample
- 10 mg/ml Test sample: 3 samples for each polymer to be tested

Pre-conditioning:

Condition and stabilize column and detectors with mobile phase (THF) until reaching working flow rate of 1 ml/min and purge viscometer and refraction index detectors, checking at the end that all signals are stable and adequate.

Chromatographic conditions:

- Column: 2 serial columns i-MBMMW-3078 (CLM1012, Viscotek)
- Delay column: medium delay (CLM9002, Viscotek)
- Column temperature 30°C
- Flux rate 1 ml/min
- Injection volume: 100 $\mu$l
- Run time: 35 minutes
- Eluent: stabilized THF (pre-heated to 30°C and under 100 rpm agitation)

System verification: Inject 100 $\mu$l of eluent and check there is no response in signals related with molecular weight determination

- Inject 100 $\mu$l of polystyrene narrow standard and check adequacy of the measurement. Repeat at least twice. Acceptance Criteria: $\pm$5% of the nominal Molecular Weight and $\pm$3% Intrinsic Viscosity declared by manufacturer standard certificate.

Calibration:

Not necessary if system verification complies and no previous chromatographic conditions are changed.
In case it would be required to calibrate:

- Inject 100 $\mu$l of polystyrene standard at least twice.
- Use first sample's data for triple calibration by creating a new multidetectors - homopolymer's method.
- Introduce into the method all the data needed for internal calibration such standard values of MW, IV, dn/dc, dA/dc and refractive index of the solvent.
- Calibrate the system as the equipment specify and save the new method.
- Check with the new method the adequacy of the measurement for the second injection of the standard.

Procedure:

Inject by triplicate 100 $\mu$l of the test sample
Polymer molecular weight measured according to the technique specified resulted in 32 KDa. According to a similar technique, inherent viscosity of the polymer resulted in a value of 0.27 dl/g. It is important to mention that inherent viscosity values correspond to those obtained with the technique described, specially related to temperature conditions and eluent used. Any change in measurement conditions mean the obtention of different values as directly depend on them.

The risperidone implantable formulation was prepared by connecting male and female syringes and moving the plungers forwards and backwards upon complete dissolution of the polymer and the formation of a homogeneous suspension of the risperidone in the polymer dissolution.

*In vivo plasma levels after intramuscular administration to Beagle dog:*

The risperidone composition of this example was intramuscularly injected to Beagle dogs weighing an average of 10 kg. The amount injected corresponded to a dose of 25 mg risperidone and the composition was intramuscularly placed in the left hind leg using a syringe with a 20G needle. Total number of dogs was 3. After injection, plasma levels were obtained at 0, 4h, 1d, 2d, 3d, 5d, 7d, 10d, 15d, 17d, 21d, 24d, 28d, 30d, 35d, 37d and 42d.

The kinetics of the plasma levels corresponding to the risperidone active moiety was evaluated by measuring both risperidone and its active metabolite 9-OH-risperidone in the plasma samples. The profile of the plasma levels of the risperidone active moiety and the AUC values calculated are shown in Figure 1. The results are expressed as the addition of risperidone plus 9-OH-risperidone concentrations (ng/ml) as the function of time, since the therapeutic activity of 9-OH-risperidone is substantially equivalent to that of risperidone.

As it can be observed in this Figure, the injection of an amount of composition equivalent to 25 mg risperidone to Beagle dogs resulted in very high control of the initial burst release followed by a slow, sustained decrease, with continuous plasma levels from day 1 onwards.

The plasma levels profile for the active moiety, as previously described, can be considered adequate as provide very low risk of having toxic plasma levels just after the injection. The adequate balance in this composition between drug solubility in the solvent and the molecular weight of the polymer in the implant (that controls the polymer precipitation process and the final structural characteristics of the implant) allows the formulation to limit the amount of risperidone that can be released in the solvent diffusion phase after the intramuscular injection.

Once the formulation is injected in the intramuscular tissue, the DMSO is rapidly dissolved in the surrounding aqueous environment. The relative increase of the polymer concentration in DMSO over the polymer solubility in the solvent leads to the formation of a polymer precipitate that entraps the risperidone that was not solubilized in the solvent. Molecular weight of the polymer has a great impact in this critical step, as too low weighed chains have delayed precipitation time compared to the chains having the weight in the adequate range. This delayed precipitation allows the drug to increase contact with the surrounding fluids towards the drug is being released. Therefore, low molecular weighed chains lead to an excessive release of risperidone after the injection and potentially to obtain toxic plasma levels on the first days after the injection. Molecular weight of the polymer also can affect the release of the drug from the intramuscularly injected implant after solvent diffusion and polymer precipitation.

Molecular weights over the specified range are not capable to maintain adequate release rates of risperidone by diffusion. Additionally, higher molecular weight chains in the intramuscular tissue require longer hydrolysis times in order to provide soluble fractions that could release the drug entrapped in the polymer matrix. A higher remaining drug content to be released could lead to the obtention of undesirably high active moiety plasma values, or plasma values after 30 days post injection that could somehow interfere with the following dose as the formulation is intended to be injected several times into the human, each 4 weeks or 30 days.

The figure 1 shows how a polymer in the 30-36 KDa region (32KDa) is capable to provide desirable in vivo plasma levels profile.

| | AUC all | AUC $_{0-7}$ days | AUC $_{7-21}$ days | AUC $_{21-last}$ |
|---|---|---|---|---|
| **Dose 2.5 mg/kg** | (h*ng/ml) 20259.70 | (h*ng/ml) 7039.78 | (h*ng/ml) 8657.52 | (h*ng/ml) 4562.40 |

**Example 2: Depot formulation with Resomer® 504 radiated to 16 KGy**

The present example shows how the polymer molecular weight can be controlled in order to have a sterile formulation with the desired in vivo release properties.

Filling solid polymer in syringes represents a real challenge in the manufacturing of injectable formulations. The polymer, manufactured as a non-sterile product, requires undergoing sterilization in order to achieve a formulation that can be injected into human beings. Probably the best way to solve this technical issue is to subject the polymer to sterilization by gamma or beta irradiation. Irradiation represents a challenging issue when used biodegradable polymers, as irradiation can disrupt the chains into fractions of smaller size. Control of the polymer molecular weight appears as again as the critical parameter to control the final characteristics of a product after a sterilization process.

However, chain size reduction by irradiation can be mathematically modelled or controlled in order to predict the final molecular weight of a polymer to be used as raw material having a molecular weight higher than desired. Therefore, once determined the fill weight of the polymer to be filled in a container (for example, the fill weight of the polymer in a syringe) and the bio-burden present in the polymer as raw material, the irradiation dose required to get the polymer sterile (as specified by ISO 11137) is selected for the required fill weight.

Then the mathematical model describing the loss of molecular weight for a certain polymer versus the irradiated dose can identify the initial molecular weight of the polymer to be used as raw material required obtaining, after the irradiation process, a polymer with the desired final molecular weight for the formulation.

As the availability of a polymer with a specific molecular weight can be somewhat limited, then we can alternatively select an available polymer with a molecular weight that is higher to what is required according to the irradiation dose identified, and then adjust the irradiation dose to a higher value in order to obtain a sterile polymer with the required molecular weight. In this example, a lactic-co-glycolic acid copolymer with 50% content of each of the two organic acid monomers and a molecular weight of 38 KDa was sterilized by beta irradiation at 16 KGy under controlled temperature and moisture conditions. The resultant polymer was characterized for its molecular weight according to the method described in example 1. Molecular weight after irradiation process was 31 KDa.

| Female 2,25 ml syringe | Ingredient | Amount (mg) |
|---|---|---|
| | Lactic-co-glycolic acid copolymer (N-capped) with 50% content of each of the two organic acid monomers and a molecular weight of 38KDa, beta-irradiated as a bulk with a 16 KGy dose achieving a final molecular weight of 31 KDa. | 50 |
| | Risperidone | 25 |
| Male 2,25 ml syringe | Ingredient | Amount (mg) |
| | Dimethyl sulfoxide | 117 |

Risperidone particle size was characterized by light scattering and provided the following distribution of particle size: $d(0,1) = 27.49$ $\mu$m, $d(0,5) = 79.90$ $\mu$m and $d(0,9) = 176.66$ $\mu$m.

Inherent viscosity of the irradiated polymer, as calculated by the technique described in example 1 was 0.27 dl/g.

The risperidone implantable formulation was prepared by connecting male and female syringes and moving the plungers forwards and backwards upon complete dissolution of the polymer and the formation of a homogeneous suspension of the risperidone in the polymer dissolution.

*In vivo plasma levels after intramuscular administration to Beagle dog:*

The risperidone composition of this example was intramuscularly injected to Beagle dogs weighing an average of 10 kg. Two cohorts were studied at two different doses: 2.5 mg/kg and 5.0 mg/kg. The composition was intramuscularly placed in the left hind leg using a syringe with a 20G needle. Total number of dogs on each cohort was 6. After injection, plasma levels were obtained at 0, 4h, 1d, 2d, 3d, 5d, 7d, 10d, 14d, 17d, 21d, 24d, 28d, 30d, 32d, 35d, 38d, 42d, 45d, 49d, 52d.

The kinetics of the plasma levels corresponding to the risperidone active moiety was evaluated by measuring both risperidone and its active metabolite 9-OH-risperidone in the plasma samples. The profile of the plasma levels of the risperidone active moiety and the AUC values calculated are shown in Figure 2. The results are expressed as the addition of risperidone plus 9-OH-risperidone concentrations (ng/ml) as the function of time, since the therapeutic activity of 9-OH-risperidone is substantially equivalent to that of risperidone. As it can be observed in this Figure, the injection of an amount of composition equivalent to 2.5 mg/kg and 5.0 mg/kg risperidone to Beagle dogs resulted again in very high control of the initial burst release followed by a slow, sustained decrease, with continuous plasma levels from day 1 onwards.

The figure 2 shows how a polymer with higher molecular weight can be adjusted to a desired molecular weight and maintain the release characteristics obtained with a non-irradiated polymer with the original molecular weight in the range of 30-36 KDa, even though small variations in the terminal end groups of the polymer chains can occur after radiation. Again, the plasma levels profile for the active moiety, as previously described, can be considered adequate as provide very low risk of having toxic plasma levels just after the injection.

| | AUC all | AUC $_{0-7}$ days | AUC $_{7-21}$ days | AUC $_{21-last}$ |
|---|---|---|---|---|
| Dose 2.5 mg/kg | (h*ng/ml) 17537.68 | (h*ng/ml) 4848.4 | (h*ng/ml) 6494.76 | (h*ng/ml) 6194.52 |
| Dose 5 mg/kg | 46924.94 | 9918.74 | 17170.8 | 19835.4 |

**Example 3: Depot formulation with Resomer® 504 radiated to 25 KGy**

This is another example that shows how the polymer molecular weight can be controlled in order to have a sterile formulation with the desired in vivo release properties.

A lactic-co-glycolic acid copolymer with 50% content of each of the two organic acid monomers and a molecular weight of 50 KDa was sterilized by beta irradiation at 25 KGy under controlled temperature and moisture conditions. The resultant polymer was characterized for its molecular weight according to the method described in example 1. Molecular weight after irradiation process was 35KDa.

| Female 2,25 ml syringe | Ingredient | Amount (mg) |
|---|---|---|
| | Lactic-co-glycolic acid copolymer (N-capped) with 50% content of each of the two organic acid monomers and a molecular weight of 50KDa, beta-irradiated as a bulk with a 25KGy dose achieving a final molecular weight of 35KDa. | 50 |
| | Risperidone | 25 |
| Male 2,25 ml syringe | Ingredient | Amount (mg) |
| | Dimethyl sulfoxide | 117 |

Risperidone particle size was characterized by light scattering and provided the following distribution of particle size: d(0,1) = 27.49 $\mu$m, d(0,5) = 79.90 $\mu$m and d(0,9) = 176.66 $\mu$m.

Inherent viscosity of the irradiated polymer, as calculated by the technique described in example 1 was 0.28 dl/g.

The risperidone implantable formulation was prepared by connecting male and female syringes and moving the plungers forwards and backwards upon complete dissolution of the polymer and the formation of a homogeneous suspension of the risperidone in the polymer dissolution.

*In vivo plasma levels after intramuscular administration to Beagle dog:*

The risperidone composition of this example was intramuscularly injected to Beagle dogs weighing an average of 10 kg. An amount of formulation equivalent to a dose of 2,5 mg/kg of risperidone was intramuscularly placed in the left hind leg using a syringe with a 20G needle. Total number of dogs was 3 After injection, plasma levels were obtained at 0, 4h, 1d, 2d, 3d, 5d, 7d, 10d, 14d, 17d, 21d, 24d, 28d, 30d, 32d, 35d, 38d, 42d, 45d, 49d, 52d.

The kinetics of the plasma levels corresponding to the risperidone active moiety was evaluated by measuring both risperidone and its active metabolite 9-OH-risperidone in the plasma samples. The profile of the plasma levels of the risperidone active moiety and the AUC values calculated are shown in Figure 3. The results are expressed as the addition of risperidone plus 9-OH-risperidone concentrations (ng/ml) as the function of time, since the therapeutic activity of 9-OH-risperidone is substantially equivalent to that of risperidone. As it can be observed in this Figure 3, the injection of an amount of composition equivalent to 2.5 mg/kg to Beagle dogs resulted again in very high control of the initial burst release followed by a slow, sustained decrease, with continuous plasma levels from day 1 onwards. Once again, the plasma levels profile for the active moiety, as previously described, can be considered adequate as provide very low risk of having toxic plasma levels just after the injection.

| | AUC all | AUC $_{0-7}$ days | AUC $_{7-21}$ days | AUC $_{21-last}$ |
|---|---|---|---|---|
| **Dose 2.5 mg/kg** | (h*ng/ml) 17734.84 | (h*ng/ml) 5134 | (h*ng/ml) 6844.08 | (h*ng/ml) 5756.76 |

**Example 4: Depot formulation with Lakeshore Biomaterials® 5050 DLG 5E radiated to 25 KGy**

This is another example that shows how the polymer molecular weight can be controlled in order to have a sterile formulation with the desired in vivo release properties.

A lactic-co-glycolic acid copolymer with 50% content of each of the two organic acid monomers and a molecular weight of 56 KDa was sterilized by beta irradiation at 25 KGy under controlled temperature and moisture conditions. The resultant polymer was characterized for its molecular weight according to the method described in example 1. Molecular weight after irradiation process was 45 KDa.

| | Ingredient | Amount (mg) |
|---|---|---|
| Female 2,25 ml syringe | Lactic-co-glycolic acid copolymer (N-capped) with 50% content of each of the two organic acid monomers and a molecular weight of 56KDa, beta-irradiated as a bulk with a 25KGy dose achieving a final molecular weight of 45KDa. | 50 |
| | Risperidone | 25 |
| Male 2,25 ml syringe | Ingredient | Amount (mg) |
| | Dimethyl sulfoxide | 117 |

Risperidone particle size was characterized by light scattering and provided the following distribution of particle size: d(0,1) = 27.49 $\mu$m, d(0,5) = 79.90 $\mu$m and d(0,9) = 176.66 $\mu$m.

Inherent viscosity of the irradiated polymer, as calculated by the technique described in example 1 was 0.28 dl/g.

The risperidone implantable formulation was prepared by connecting male and female syringes and moving the plungers forwards and backwards upon complete dissolution of the polymer and the formation of a homogeneous suspension of the risperidone in the polymer dissolution.

*In vivo plasma levels after intramuscular administration to White New Zealand rabbits*

The risperidone composition of this example was intramuscularly injected to White New Zealand rabbits weighing an average of 3 kg. An amount of formulation equivalent to a dose of 5 mg/kg of risperidone was intramuscularly placed in the left hind leg using a syringe with a 20G needle. Total number of rabbits was 3. After injection, plasma levels were obtained at 0, 4h, 1d, 3d, 5d, 7d, 10d, 14d, 17d, 21d, 24d, 28d, 31d.

The kinetics of the plasma levels corresponding to the risperidone active moiety was evaluated by measuring both risperidone and its active metabolite 9-OH-risperidone in the plasma samples. The profile of the plasma levels of the risperidone active moiety and the AUC values calculated are shown in Figure 4. The results are expressed as the addition of risperidone plus 9-OH-risperidone concentrations (ng/ml) as the function of time, since the therapeutic activity of 9-OH-risperidone is substantially equivalent to that of risperidone. As it can be observed in this Figure 4, the injection of an amount of composition equivalent to 5 mg/kg to White New Zealand rabbits resulted again in high control of the initial burst release followed by a slow, sustained decrease, with continuous plasma levels from day 1 onwards. Once again, the plasma levels profile for the active moiety, as previously described, can be considered adequate as provide very low risk of having toxic plasma levels just after the injection.

In this example, in which rabbits were used as experimental model instead of dogs, it is not applicable the same consideration as an "adequate plasma level profile" than in Beagle dog. Rabbits exhibit a higher body temperature than Beagle dogs, about 40°C instead of about 37-38°C. Rabbit model shows and accelerated implant degradation, and in consequence an accelerated risperidone release profile, in comparison to dog and human because that higher body temperature promotes a faster polymer degradation. This accelerated degradation of polymer does not affect the first stage of drug release profile during which implant is not degrading yet, but it leads to a faster second stage during drug is released by diffusion and degradation and it leadsd to a consequently shorter duration term. For that reason, in case of White New Zealand rabbit, an "adequate plasma level profile" is considered as not more than the 35% of the AUC of the active moiety (risperidone + 9-OH risperidone plasma concentrations) occurring between the moment of the injection until day 7 (included), between 35% and 55% of the AUC of the active moiety occurring from day 7 and until 17 (included), and not more than 35% of the AUC of the active moiety occurring after day 17.

| | AUC all | $AUC_{0-7}$ days | $AUC_{7-17}$ days | $AUC_{17-last}$ |
|---|---|---|---|---|
| Dose 5 mg/kg | (h*ng/ml) 122924.61 | (h*ng/ml) 4162.63 | (h*ng/ml) 6521.64 | (h*ng/ml) 2240.34 |

**Example 5: Depot formulation with Resomer® 504 radiated to 25 KGy**

In this example, a lactic-co-glycolic acid copolymer with 50% content of each of the two organic acid monomers and a molecular weight of 38 KDa was sterilized by beta irradiation at 25 KGy under controlled temperature and moisture conditions. The resultant polymer was characterized for its molecular weight according to the method described in example 1. Molecular weight after irradiation process was 28 KDa.

| | Ingredient | Amount (mg) |
|---|---|---|
| Female 2,25 ml syringe | Lactic-co-glycolic acid copolymer (N-capped) with 50% content of each of the two organic acid monomers and a molecular weight of 38KDa, beta-irradiated as a bulk with a 25 KGy dose achieving a final molecular weight of 28 KDa. | 50 |
| | Risperidone | 25 |
| Male 2,25 ml syringe | Ingredient | Amount (mg) |
| | Dimethyl sulfoxide. | 117 |

Risperidone particle size was characterized by light scattering and provided the following distribution of particle size: d(0,1) = 27.49 $\mu$m, d(0,5) = 79.90 $\mu$m and d(0,9) = 176.66 $\mu$m.

Inherent viscosity of the irradiated polymer, as calculated by the technique described in example 1 was 0.25 dl/g.

The risperidone implantable formulation was prepared by connecting male and female syringes and moving the plungers forwards and backwards upon complete dissolution of the polymer and the formation of a homogeneous suspension of the risperidone in the polymer dissolution.

*In vivo plasma levels after intramuscular administration to Beagle dog:*

The risperidone composition of this example was intramuscularly injected to Beagle dogs weighing an average of 10 kg. An amount of formulation equivalent to a dose of 2,5 mg/kg of risperidone was intramuscularly placed in the left hind leg using a syringe with a 20G needle. Total number of dogs per cohort was 3. After injection, plasma levels were obtained at 0, 4h, 1d, 2d, 3d, 5d, 7d, 10d, 14d, 17d, 21d, 24d, and 28d.

The kinetics of the plasma levels corresponding to the risperidone active moiety was evaluated by measuring both risperidone and its active metabolite 9-OH-risperidone in the plasma samples. The profile of the plasma levels of the risperidone active moiety and the AUC values calculated are shown in Figure 5. The results are expressed as the addition of risperidone plus 9-OH-risperidone concentrations (ng/ml) as the function of time, since the therapeutic activity of 9-OH-risperidone is substantially equivalent to that of risperidone. As it can be observed in this Figure 5, the injection of an amount of composition equivalent to 2.5 mg/kg to Beagle dogs resulted in a plasma values profile that is different to the previous formulations tested. The figure shows how the formulation provides higher active moiety plasma values, probably due to a reduced control of the polymer on the release of the drug once the formulation has been injected. A reduced molecular weight could also lead to increased uptake of water throughout the time, leading to greater release of the risperidone by diffusion and to a reduced time in which the polymer hydrolizes into smaller sized soluble fractions. It is noticeable to mention that this different polymer behaviour could be obtained with a polymer with a molecular weight only 3 KDa less compared to example 2.

| | AUC all | AUC $_{0-7}$ days | AUC $_{7-21}$ days | AUC $_{21-last}$ |
|---|---|---|---|---|
| **Dose 2.5 mg/kg** | (h*ng/ml) 21702.82 | (h*ng/ml) 10021.78 | (h*ng/ml) 9662.28 | (h*ng/ml) 2018.76 |

**Example 6: Depot formulation with Resomer® 503 radiated to 15 KGy**

In this example, a lactic-co-glycolic acid copolymer with 50% content of each of the two organic acid monomers and a molecular weight of 32 KDa was sterilized by beta irradiation at 15 KGy under controlled temperature and moisture conditions. The resultant polymer was characterized for its molecular weight according to the method described in example 1. Molecular weight after irradiation process was 28.3 KDa.

| | Ingredient | Amount (mg) |
|---|---|---|
| Female 2,25 ml syringe | Lactic-co-glycolic acid copolymer (N-capped) with 50% content of each of the two organic acid monomers and a molecular weight of 32 KDa, beta-irradiated as a bulk with a 15 KGy dose achieving a final molecular weight of 28.3 KDa. | 50 |
| | Risperidone | 25 |

(continued)

| Male 2,25 ml syringe | Ingredient | Amount (mg) |
|---|---|---|
| | Dimethyl sulfoxide | 117 |

Risperidone particle size was characterized by light scattering and provided the following distribution of particle size: d(0,1) = 27.49 $\mu$m, d(0,5) = 79.90 $\mu$m and d(0,9) = 176.66 $\mu$m.

Inherent viscosity of the irradiated polymer, as calculated by the technique described in example 1 was 0.25 dl/g.

The risperidone implantable formulation was prepared by connecting male and female syringes and moving the plungers forwards and backwards upon complete dissolution of the polymer and the formation of a homogeneous suspension of the risperidone in the polymer dissolution.

*In vivo plasma levels after intramuscular administration to Beagle dog:*

The risperidone composition of this example was intramuscularly injected to Beagle dogs weighing an average of 12.5 kg. An amount of formulation equivalent to a dose of 25 mg of risperidone was intramuscularly placed in the left hind leg using a syringe with a 20G needle. Total number of dogs per cohort was 3. After injection, plasma levels were obtained at 0, 4h, 1d, 2d, 3d, 5d, 7d, 10d, 14d, 17d, 21d, 24d, and 28d.

The kinetics of the plasma levels corresponding to the risperidone active moiety was evaluated by measuring both risperidone and its active metabolite 9-OH-risperidone in the plasma samples. The profile of the plasma levels of the risperidone active moiety and the AUC values calculated are shown in Figure 6. The results are expressed as the addition of risperidone plus 9-OH-risperidone concentrations (ng/ml) as the function of time, since the therapeutic activity of 9-OH-risperidone is substantially equivalent to that of risperidone. As it can be observed in this Figure, the result was similar to what it was obtained in example 5. This time, the reduction of molecular weight from the original is only 3.7 KDa, smaller to that observed in examples 2, 3 and 4. This is important to notice, as it could had been thought that the more irregular plasma levels profile obtained in example 5 was mostly due to an intense reduction in the polymer molecular weight, potentially leading to an increased heterogeneity in the distribution of the different sized chains. This example shows how the molecule can be tailored to a specific molecular weight distribution and to obtain similar plasma levels profile.

| | AUC all | AUC $_{0-7}$ days | AUC $_{7-21}$ days | AUC $_{21-last}$ |
|---|---|---|---|---|
| Dose 2 mg/kg | (h*ng/ml) 13245.14 | (h*ng/ml) 6873.74 | (h*ng/ml) 5371.44 | (h*ng/ml) 999.96 |

**Example 7: Depot formulation with Resomer® 504 without radiation**

| In this example, a lactic-co-glycolic acid copolymer with 50% content of each of the two organic acid monomers and a molecular weight (according to method described in example 1) of 48KDa was used.Female 2,25 ml syringe | Ingredient | Amount (mg) |
|---|---|---|
| | Lactic-co-glycolic acid copolymer (N-capped) with 50% content of each of the two organic acid monomers and a molecular weight of 48 KDa. | 50 |
| | Risperidone | 25 |
| Male 2,25 ml syringe | Ingredient | Amount (mg) |
| | Dimethyl sulfoxide | 117 |

Risperidone particle size was characterized by light scattering and provided the following distribution of particle size: d(0,1) = 27.49 $\mu$m, d(0,5) = 79.90 $\mu$m and d(0,9) = 176.66 $\mu$m.

Inherent viscosity of the irradiated polymer, as calculated by the technique described in example 1 was 0.33 dl/g.

The risperidone implantable formulation was prepared by connecting male and female syringes and moving the plungers forwards and backwards upon complete dissolution of the polymer and the formation of a homogeneous suspension of the risperidone in the polymer dissolution.

*In vivo plasma levels after intramuscular administration to Beagle dog:*

The risperidone composition of this example was intramuscularly injected to Beagle dogs weighing an average of 12.5 kg. An amount of formulation equivalent to a dose of 25 mg of risperidone was intramuscularly placed in the left hind leg using a syringe with a 20G needle. Total number of dogs per cohort was 3. After injection, plasma levels were obtained at 0, 4h, 1d, 2d, 3d, 5d, 7d, 10d, 14d, 17d, 21d, 24d, and 28d.

The kinetics of the plasma levels corresponding to the risperidone active moiety was evaluated by measuring both risperidone and its active metabolite 9-OH-risperidone in the plasma samples. The profile of the plasma levels of the risperidone active moiety and the AUC values calculated are shown in Figure 7. The results are expressed as the addition of risperidone plus 9-OH-risperidone concentrations (ng/ml) as the function of time, since the therapeutic activity of 9-OH-risperidone is substantially equivalent to that of risperidone. The figure shows the effect of using a polymer with a molecular weight that is higher than the range valid for this formulation. Initial plasma values do not change in a significant way, as risperidone solubility in the DMSO and DMSO diffusion towards surrounding liquids are the major factors controlling the release of risperidone from the implant. Then, it can be seen how a reduced release by diffusion leads to a reduction in plasma levels of active moiety. The higher molecular weight chains of the polymer also increases the time required to release the risperidone by the formation of reduced molecular weight soluble polymer molecules by hydrolysis. This fact is detected in the active moiety plasma levels profile as a delayed peak.

|  | **AUC all** | **AUC $_{0-7}$ days** | **AUC $_{7-21}$ days** | **AUC $_{21\text{-last}}$** |
|---|---|---|---|---|
| Dose 2mg/kg | (h*ng/ml) 15601.88 | (h*ng/ml) 2902.28 | (h*ng/ml) 2935.32 | (h*ng/ml) 9764.28 |

## Example 8: Depot formulation with Resomer® 504 radiated to 25 KGy

The current example demonstrates the concept is also valid to achieve an intramuscularly injectable paliperidone formulation suitable to be administered once each 4 weeks.

A lactic-co-glycolic acid copolymer with 50% content of each of the two organic acid monomers and a molecular weight of 50 KDa was sterilized by beta irradiation at 25KGy under controlled temperature and moisture conditions. The resultant polymer was characterized for its molecular weight according to the method described in example 1. Molecular weight after irradiation process was 35KDa.

| Female 2,25 ml syringe | Ingredient | Amount (mg) |
|---|---|---|
|  | Lactic-co-glycolic acid copolymer (N-capped) with 50% content of each of the two organic acid monomers and a molecular weight of 50 KDa, beta-irradiated as a bulk with a 25KGy dose achieving a final molecular weight of 35KDa. | 50 |
|  | Paliperidone | 25 |

| Male 2,25 ml syringe | Ingredient | Amount (mg) |
|---|---|---|
|  | Dimethyl sulfoxide | 117 |

Paliperidone particle size was characterized by light scattering and provided the following distribution of particle size: d(0,1) = 17.41 $\mu$m, d(0,5) = 51.61 $\mu$m and d(0,9) = 175.32 $\mu$m.

Inherent viscosity of the irradiated polymer, as calculated by the technique described in example 1 was 0.28 dl/g.

The paliperidone implantable formulation was prepared by connecting male and female syringes and moving the plungers forwards and backwards upon complete dissolution of the polymer and the formation of a homogeneous suspension of the paliperidone in the polymer dissolution.

*In vivo plasma levels after intramuscular administration to Beagle dog:*

The paliperidone composition of this example was intramuscularly injected to Beagle dogs weighing an average of 10 kg. An amount of formulation equivalent to a dose of 1.5 mg/kg of risperidone was intramuscularly placed in the left hind leg using a syringe with a 20G needle. Total number of dogs per cohort was 3. After injection, plasma levels were obtained at 0, 4h, 1d, 2d, 3d, 5d, 7d, 10d, 14d, 17d, 21d, 24d, 28d, 31d, 35d, 38d, 42d, 45d, 49d, 52d, 56d, 59d, 63d, 70d, 77d.

The kinetics of the plasma levels corresponding to the paliperidone was evaluated and and the AUC values calculated are shown in Figure 8. The results are expressed as paliperidone concentrations (ng/ml) as the function of time. As it can be observed in this Figure, the injection of an amount of composition equivalent to 1.5 mg/kg to Beagle dogs resulted again in very high control of the initial burst release followed by a continuous paliperidone plasma level profile during 59 days. The difference in the release properties compared to the same risperidone formulation can be related to different pKa values of both drugs that can affect the in vivo biodegradation properties of the polymer and produce release of the drug over a longer period of time. The formulation tested demonstrates the feasibility of the composition to obtain a paliperidone formulation that can provide prolonged release of paliperidone during an entire month and can be administered each 4 weeks or even each longer times.

|  | **AUC all** | **AUC $_{0-7}$ days** | **AUC $_{7-21}$ days** | **AUC $_{21\text{-last}}$** |
|---|---|---|---|---|
| **Dose 1.5** mg/kg | (h*ng/ml) 8636.94 | (h*ng/ml) 1160.34 | (h*ng/ml) 3630.84 | (h*ng/ml) 3845.76 |

## Claims

1.  An injectable intramuscular depot composition suitable for forming an *in situ* solid implant in a body, comprising a drug which is risperidone and/or any pharmaceutically acceptable salt thereof in any combination, a biocompatible copolymer based on lactic and glycolic acid having a monomer ratio of lactic to glycolic acid of from 45:55 to 55:45 and DMSO as solvent, wherein the composition releases the drug with an immediate onset of action and continuously for at least 4 weeks and wherein the composition has a pharmacokinetic profile *in vivo* that makes it suitable to be administered each 4 weeks or even longer periods and wherein the biocompatible copolymer has a molecular weight in the range of between 30 and 46 kDa and an inherent viscosity in the range of between 0.25-0.31 dl/g $\pm$10%, wherein the composition is for use in the treatment of schizophrenia or bipolar disorders in the human body and is **characterised by** the administration of a first intramuscular dose of between 75 to 200 mg of the composition at day 0, followed by a second dose in the range of 25 to 200 mg between days 28-35, and then subsequent 4 weekly doses.

2.  The composition for use of claim 1, wherein the biocompatible copolymer is gamma or beta irradiated in the dose range of 10-30 KGy measured at a temperature between -40°C and +35°C to adjust its molecular weight to a range between 30 and 46 kDa and its inherent viscosity to a range of between 0.25-0.31 dl/g $\pm$10%.

3.  The composition for use of claims 1 or 2, wherein the particle size distribution of the drug is:

    - less than 10% particles smaller than 10 microns;
    - less than 10% particles larger than 225 microns, and
    - a d0.5 value in the range of 40-130 microns.

4.  The composition for use according to any one of claims 1 to 3, wherein the drug / (polymer+drug) mass ratio is about 33%.

5.  The composition for use according to any one of claims 1 to 4, wherein the content of drug is about 13% w/w of total formulation and the viscosity of the solution comprising the polymer and the DMSO is in the range of 1.5-2.1 P.a.s $\pm$10%.

6.  A pharmaceutical kit suitable for the in situ formation of a biodegradable implant in a body comprising the composition for use of any one of claims 1-5, wherein the drug and the biocompatible polymer are contained in a first container, and the solvent is contained in a second, separate container.

7.  The pharmaceutical kit according to claim 6, wherein at least one of the first and second containers is a syringe, a vial, a device or a cartridge, either disposable or not.

**Patentansprüche**

1. Injizierbare intramuskuläre Depot-Zusammensetzung, die zur Bildung eines in situ festen Implantats in einem Körper geeignet ist, umfassend ein Arzneimittel, das Risperidon und/oder ein pharmazeutisch verträgliches Salz davon in einer beliebigen Kombination ist, ein biokompatibles Copolymer auf der Basis von Milchsäure und Glykolsäure mit einem Monomerverhältnis von Milchsäure zu Glykolsäure von 45:55 bis 55:45 und DMSO als Lösungsmittel, wobei die Zusammensetzung das Arzneimittel mit sofortigem Wirkungseintritt und kontinuierlich über mindestens 4 Wochen freisetzt und wobei die Zusammensetzung ein pharmakokinetisches Profil in vivo aufweist, das es zur Verabreichung alle 4 Wochen oder noch länger geeignet macht und wobei das biokompatible Copolymer ein Molekulargewicht im Bereich zwischen 4,9816206e-23 kg (30 kDa) und 7,63848492e-23 kg (46 kDa) und eine inhärente Viskosität im Bereich zwischen 0,25-0,31 dl/g ±10 % aufweist, wobei die Zusammensetzung zur Behandlung von Schizophrenie oder bipolaren Störungen im menschlichen Körper verwendet wird und durch die Verabreichung einer ersten intramuskulären Dosis von 75 bis 200 mg der Zusammensetzung an Tag 0, gefolgt von einer zweiten Dosis im Bereich von 25 bis 200 mg zwischen den Tagen 28 bis 35 und anschließend 4 wöchentlichen Dosen gekennzeichnet ist.

2. Zusammensetzung zur Verwendung nach Anspruch 1, wobei das biokompatible Copolymer Gamma- oder Beta-bestrahlt im Dosisbereich von 10-30 KGy ist, gemessen bei einer Temperatur zwischen -40 °C und +35 °C, um sein Molekulargewicht auf einen Bereich zwischen 4,9816206e-23 kg (30 kDa) und 7,63848492e-23 kg (46 kDa) und seine inhärente Viskosität auf einen Bereich zwischen 0,25-0,31 dl/g ±10 % einzustellen.

3. Zusammensetzung zur Verwendung nach Anspruch 1 oder 2, wobei die Partikelgrößenverteilung des Arzneimittels

   - weniger als 10 % Partikel, die kleiner als 10 Mikrometer sind;
   - weniger als 10 % Partikel, die größer als 225 Mikrometer sind, und
   - einen Wert von d0,5 im Bereich von 40--130 Mikrometer aufweist.

4. Zusammensetzung zur Verwendung nach einem der Ansprüche 1 bis 3, wobei das Massenverhältnis von Arzneimittel / (Polymer+Arzneimittel) etwa 33 % beträgt.

5. Zusammensetzung zur Verwendung nach einem der Ansprüche 1 bis 4, wobei der Wirkstoffgehalt etwa 13 Gew.-% der Gesamtformulierung beträgt und die Viskosität der Lösung, die das Polymer und das DMSO umfasst, im Bereich von 1,5-2,1 p.a.s ±10 % liegt.

6. Pharmazeutisches Kit, das für die in-situ-Bildung eines biologisch abbaubaren Implantats in einem Körper geeignet ist, umfassend die Zusammensetzung zur Verwendung nach einem der Ansprüche 1 bis 5, wobei das Arzneimittel und das biokompatible Polymer in einem ersten Behälter enthalten sind und das Lösungsmittel in einem zweiten, separaten Behälter enthalten ist.

7. Pharmazeutisches Kit nach Anspruch 6, wobei mindestens einer der ersten und zweiten Behälter eine Spritze, ein Fläschchen, eine Vorrichtung oder eine Patrone ist, die Einwegprodukte oder keine Einwegprodukte sein können.

**Revendications**

1. Composition de dépôt intramusculaire injectable appropriée pour former un implant solide in situ dans un corps, comprenant un médicament qui est la rispéridone et/ou tout sel pharmaceutiquement acceptable de celle-ci dans une combinaison quelconque, un copolymère biocompatible à base d'acide lactique et glycolique ayant un rapport de monomères de l'acide lactique à l'acide glycolique de 45:55 à 55:45 et du DMSO comme solvant, la composition libérant le médicament avec un début d'action immédiat et en continu pendant au moins 4 semaines et la composition ayant un profil pharmacocinétique in vivo qui la rend appropriée pour être administrée toutes les 4 semaines ou même pendant des périodes plus longues et le copolymère biocompatible ayant un poids moléculaire compris entre 4,9816206e-23 kg (30 kDa) et 7,63848492e-23 kg (46 kDa) et une viscosité inhérente comprise entre 0,25 et 0,31 dl/g ±10 %, la composition étant destinée à être utilisée dans le traitement de la schizophrénie ou des troubles bipolaires dans le corps humain et étant **caractérisée par** l'administration d'une première dose intramusculaire comprise entre 75 et 200 mg de la composition au jour 0, puis d'une deuxième dose comprise entre 25 et 200 mg entre les jours 28 et 35, puis de 4 doses hebdomadaires ultérieures.

**2.** Composition destinée à être utilisée selon la revendication 1, dans laquelle le copolymère biocompatible est irradié gamma ou bêta dans la plage de doses de 10 à 30 KGy mesurée à une température comprise entre -40 °C et +35 °C pour ajuster son poids moléculaire à une plage comprise entre 4,9816206e-23 kg (30 kDa) et 7,63848492e-23 kg (46 kDa) et sa viscosité inhérente à une plage comprise entre 0,25 et 0,31 dl/g ±10 %.

**3.** Composition destinée à être utilisée selon les revendications 1 ou 2, dans laquelle la distribution granulométrique du médicament est

- inférieure à 10 % de particules de moins de 10 microns ;
- inférieure à 10 % de particules de plus de 225 microns, et
- une valeur d'0,5 dans la plage de 40 à 130 microns.

**4.** Composition destinée à être utilisée selon l'une quelconque des revendications 1 à 3, dans laquelle le rapport massique médicament/(polymère+médicament) est d'environ 33 %.

**5.** Composition destinée à être utilisée selon l'une quelconque des revendications 1 à 4, dans laquelle la teneur en médicament est d'environ 13 % p/p de la formulation totale et la viscosité de la solution comprenant le polymère et le DMSO est comprise entre 1,5 et 2,1 P.a.s ±10 %.

**6.** Trousse pharmaceutique appropriée pour la formation in situ d'un implant biodégradable dans un corps comprenant la composition destinée à être utilisée selon l'une quelconque des revendications 1 à 5, dans laquelle le médicament et le polymère biocompatible sont contenus dans un premier récipient, et le solvant est contenu dans un second récipient séparé.

**7.** Trousse pharmaceutique selon la revendication 6, dans laquelle au moins l'un des premier et second récipients est une seringue, un flacon, un dispositif ou une cartouche, jetable ou non.

Figure 1

Figure 2

Figure 3

Figure 4

Figure 5

Figure 6

Figure 7

Figure 8

Figure 9

Figure 10

**Figure 11**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 200617537 A
- WO 2008128436 A
- WO 200915828 A
- US 5965168 A
- WO 02008153611 A
- EP 2234617 A
- US 2009163519 A
- WO 201142453 A
- WO 2011151355 A

### Non-patent literature cited in the description

- IUPAC. Basic definitions of terms relating to polymers 1974. *Pure Appl. Chem.*, 1974, vol. 40, 477-491